# EUROPEAN PATENT SPECIFICATION

(11) **EP 1 089 693 B1**
(45) Date of publication and mention of the grant of the patent: **19.03.2003**
(21) Application number: 99927379.0
(22) Date of filing: 11.06.1999
(51) Int. Cl.: A61F 13/15, A61F 5/451

(54) **FAECAL COLLECTOR WITH IMPROVED ADHESIVE FLANGE ATTACHMENT MEANS**
FÄKALIENAUFNAHMEBEHÄLTER MIT EINE KLEBSTOFFLASCHE ZUR BEFESTIGUNG AN DER HAUT
COLLECTEUR FECAL A MOYEN D'ATTACHE DOTE D'UN BORD ADHESIF AMELIORE

(30) Priority: 26.06.1998 WO PCT/US98/13290
(43) Date of publication of application: 11.04.2001
(73) Proprietor: THE PROCTER & GAMBLE COMPANY, Cincinnati, Ohio 45202 (US)
(72) Inventor: PALUMBO, Gianfranco, D-65760 Eschborn (DE); D'ACCHIOLI, Vincenzo, D-65779 Kelkheim am Taunus (DE)
(74) Representative: Veronese, Pancrazio
(86) International application number: US9912960
(87) International publication number: WO00000132

(56) References cited:
- EP-A- 0 753 290
- GB-A- 1 078 588
- GB-A- 1 092 274
- GB-A- 2 116 849
- US-A- 3 577 989
- PATENT ABSTRACTS OF JAPAN vol. 096, no. 009, 30 September 1996 (1996-09-30) & JP 08 117261 A (BEPPU YOSHIO), 14 May 1996 (1996-05-14)

## Description

### Field of the Invention

The present invention relates to disposable human waste management devices such as urine management devices and faecal management devices for babies, children or adults to be attached to the skin of the wearer. The device has an improved attachment means to the skin of the wearer so as to facilitate easy application and removal of the device from the wearer without pain, whilst ensuring maintenance of the device in the desired position, particularly on wet and moist skin for the entire period of wear, including circumstances or periods of wear during which the wearer is active, i.e. not bedridden.

### Background of the Invention

Urine and faecal management devices are known articles of manufacture that are designed to be worn principally by incontinence sufferers and in particular by bedridden patients. Such faecal management devices are attached to the anal region, natural or artificial, of the wearer and or the uro genital region and are intended to entrap and immediately contain urine faecal material and other bodily discharges. Such devices as they are mostly known today are constituted of a relatively long and narrow tube, at one extremity of which an aperture and a skin attachment device, which can be adhesive are provided.

Due to their typical elongated shape and dimensions, such devices can readily twist around the thighs of the wearers and/or can cause the formation of folds and kinks in the devices themselves. Such occurrences naturally affect the storage capacity of the device and may result in unintentional detachment of the device from the wearer during use, leading to undesirable and distressing consequences both for the wearer and carer.

Such bags are disclosed in for example the following documents:

US 3,577,989, which details a disposable elimination-trapping bag for incontinence sufferers including a container member having an open-top portion, and a flange secured to the container member around the open-top portion. The flange may include a layer of adhesive on its surface as a means of attachment of the bag to the wearer or alternatively discloses the use of elastic straps to attach the bag to the wearer. US 4,784,656,describes a receptacle for collecting faecal matter from incontinence sufferers. The faecal collector comprises a gasket, conduit means or a cylinder and a receptacle; the receptacle and conduit means are each formed from two sheets of odour barrier thermoplastic film that are heat sealed along their side edges, respectively and the side surface of the gasket is coated with a layer of pressure sensitive, water resistant, medically approved adhesive; GB 2 152 387, teaches a faecal collector for incontinence sufferers comprising a collection bag and a ring, which is provided with a pressure sensitive water resistant adhesive. The faecal collector comprises a pair of panels of thermoplastic sheet material joined at their margins to define an elongate bag having an opening at one end.; SE 8 104 934, which discloses an oblong bag made from a thin, flexible and fluid tight material. The collecting bag comprises an inlet portion and a bottom portion at an angle of 120 degrees to the longitudinal direction of the inlet portion. The bag is so designed as to enable it to assume an advantageous position along the thigh of the person when in use. GB 1 078 588 describes a urine collector comprising a liquid proof bag of tube like configuration having an opening surrounded by an attachment means in the form of an adhesive bearing material.

Other types of faecal management bags having a flatter shape are known from EP 245 064. EP 245 064 discloses bags having a front and a rear wall, the front wall containing the aperture and attachment means to the body. The attachment means is a skin compatible water resistant material such as a hydrocolloid and a water insoluble viscose elastic binder. The general shape of the front and rear wall is rectangular, the width of the bag being relatively short in comparison to the length thereof.

Patent application EP 753290 also discloses a fecal management bag having a patch for attachment to the skin provided with a hydrocolloid-type adhesive.

The wearing conditions of such devices will naturally depend on the nature of the wearer; when the wearer is active, such as a baby or a child, or an incontinent adult who. is not bedridden, the wearing conditions for the bag become much more stressed and the risk of detachment of the bag will increase substantially, due to the movement of the wearer and the pressure from the wearer's body, if the containment properties are not optimum; i.e. there is a likelihood that the material, once excreted and contained in the bag, will exert pressure which may result in the unintentional detachment of the bag and undesirable consequences for the wearer and the carer. Hence, it is critical that the devices are securely attached to the wearer and do not become unintentionally unattached during use.

On the other hand, the adhesive must have a skin compatible composition and not be harsh or aggressive towards the skin or cause skin irritation or inflammation. Also it is preferred if the adhesive is compliant with the skin of the wearer such that maximum skin surface contact between the adhesive and the skin is achieved. Moreover, it is also desirable to provide an adhesive such that the device can be readily removed from the wearer, without the wearer experiencing any unacceptable pain level. This is particularly important under circumstances, where the device is misplaced, and removal and reapplication of the device once or even a number of times is required. However, on the other hand the desired level of adhesion, albeit painless should of course also be maintained during such multiple applications of the device.

The problem of the achieving the desired adhesion level is further exacerbated under wet skin conditions. Typically, prior to the: placement of the device, the skin is cleaned and is usually as a result moist. The currently available adhesives, such as hydrocolloids, however often do not immediately strongly adhere to the skin and may need to be held in place until sufficient minimum adhesion occurs. Moreover, the overall adhesive ability of such adhesives tends to be significantly reduced on wet skin surfaces per se, so that the device will typically not remain attached to the skin during wearer if any pressure is exerted onto the device, for example by the movement of the wearer or during the defaecation process.

Moist and wet skin however is not just a problem which is prevalent at the device application stage, as a significant amount of moisture is also generated during the use of the device from the wearer by perspiration and from the material contained within the bag. The resulting humid environment naturally further increases when the device is utilised in combination with a diaper. Under such circumstances current adhesives typically cannot absorb this moisture and again the adhesive strength is reduced to such an extent that the device will often become detached under exertion of pressure during wear. It is hence very important to provide an adhesive which maintains its adhesive strength on wet skin.

None of the prior art in the field of faecal management bags however even recognises or addresses the problem of providing these devices with such an attachment means which meets these criteria.

The prior art in the general field of adhesives for attachment to the skin is however more developed in the field of articles such as band-aids, plasters and bandages. These articles are, however, typically applied in an emergency situation, where for example, a cut into the skin of the wearer has occurred and absorption of the body liquids emanating from a wound is desired. In this context performance aspects of the article such as easy application and use of the product, comfortable wear as well as painless removal, and discreteness are subordinate, to criteria such as sterility, healing support, and mechanical protection of the wound.

The use of such adhesives for absorbent articles for the absorption of body liquids which naturally emanate from the body in the absence of any wounds such as for example sanitary napkins, and diapers have also been disclosed, for example in US statutory invention registration H1602 or WO 96/33683 and WO 95/16424. The latter discloses sanitary articles having a topical adhesive which is applied on the wearer facing side of a sanitary napkin along the entire periphery.

WO 96/13238 discloses a topical adhesive which is described in terms of frequency dependency. European Patent Application EP-638 303 discloses the use of a topical adhesive on side cuffs of sanitary napkins in order to keep the cuffs in an upright position. Swiss publication CH-643730 discloses the use of a very long sanitary napkin having chamfered outer edges with a topical adhesive at the four corners of the outer edges in order to provide a topical adhesive area well outside the region of pubic hair growth.

However all of these disclosures typically discuss the adhesive only in general terms or concentrate on the area of application of the adhesive to the article. The nature of adhesive per se other than the basic physical requirements such as pressure sensitivity are not discussed in particular with reference to the chemical composition.

From the field of urinary management devices it is known, for example from WO 92/11825, to provide a urinary incontinence pad having a resilient body the exterior surface of which is applied with a layer of adhesive such as a hydrophilic hydrogel adhesive. However urinary absorbent devices have to meet entirely different functional criteria than the devices of the present invention. In particular, faecal management devices contain solid or semi solid waste which can readily move within the confines of the bag and exert pressure upon the orifice and thereby cause dis-attachment of the device from the wearer. In contrast such urinary pads can readily absorb the liquid such that it will not flow out of the absorbent and therefore such devices are not designed to be able to withstand the pressures commonly exerted within a faecal management device. Similarly US 4 699 146 discloses hydrophilic elastomeric pressure sensitive adhesives for use with an electrosurgical return electrode and supportive web like substrates such as ostomy appliances.

Hence there still exists a need to provide a human waste management device for collection of excreted matter from the natural anal orifice and/or uro genital area having an adhesive for the secure attachment and painless removal of the device from the skin of the wearer and it is thus an object of the present invention to provide such as device.

It is another objective of the present invention to provide an adhesive that exhibits an ability to adhere to skin upon reapplication, particularly multiple reapplication for example when the device is misplaced is maintained, whilst still allowing painless removal. It is yet another objective to provide an adhesive which does not leave residues on the skin after removal.

It is yet a further objective of the present invention that the adhesive will adhere to moist or wet skin, independent of whether this is direct application of the device onto wet skin, or moisture which is generated on the skin surface during the wearing period of the device.

It has now been found that the above drawbacks will be substantially alleviated by providing the flange of the device with an adhesive as defined hereinafter. In a particularly preferred embodiment of the present invention, the overall performance of the devices is further improved, if the bags are provided with a particular configuration, thus allowing the utilisation of the devices for a number of wearer groups such as babies, children and active adult incontinence sufferers, in addition to bedridden adult incontinence suffers.

In another aspect of the present invention, the faecal management device with its specific adhesive can be advantageously used in combination with a reusable underwear garment or preferably with a disposable diaper.

### Summary of the Invention

The present invention relates to a human waste management device such as a urine management device or a faecal management device (10) for collecting excreted matter from the natural anal orifice and/or uro genital region, said device comprising a bag (11), said bag (11) having an aperture (21) defined by a flange (12) surrounding said aperture (21); said flange (12) having a garment facing surface (22) and a wearer facing surface (23), wherein said wearer facing surface (23) of said flange (12) comprises an adhesive (20) wherein said adhesive (20) is a substantially water insoluble pressure sensitive adhesive comprising a polymer which forms a 3-dimensional matrix, and comprises less than 10% hydrocolloids, for adhesive attachment of said device to the perianal area of wearer.

### Brief description of the drawings

It is believed that the invention will be better understood from the foregoing description in conjunction with the accompanying drawings in which:
Figure 1 is a perspective view of a faecal management device in accordance with the present invention.
Figure 2 shows a perspective view of the faecal management device in conjunction with a disposable diaper; and
Figure 3 is a partially cut-away perspective view of a disposable diaper embodying the present invention.
Figure 4 is a plan view of a disposable urine management device of the present invention.

### Detailed description of the Invention

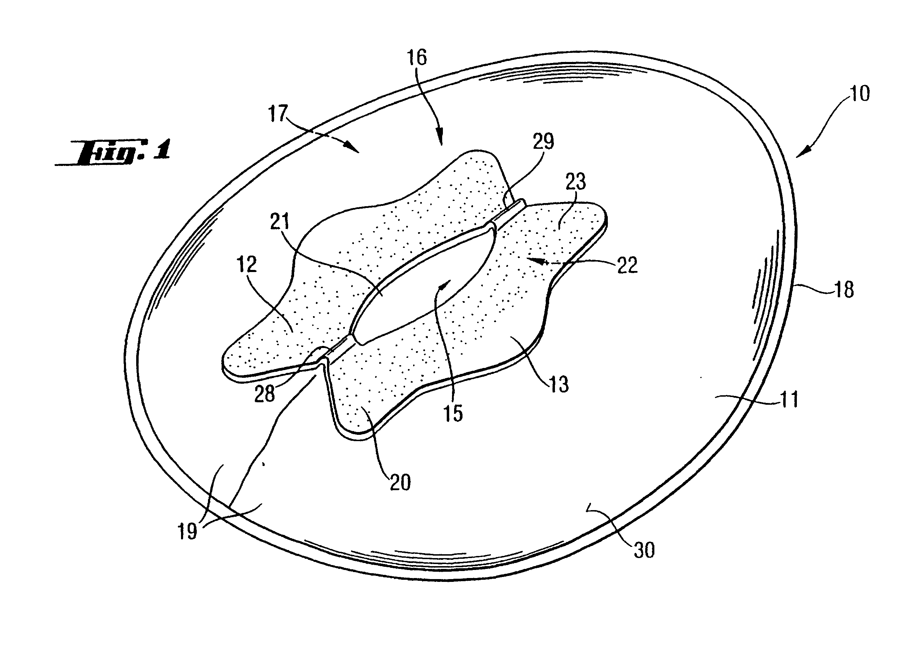

According to the present invention any disposable human waste management device such as a urine management device or a faecal management device known in the art for collecting faecal excreted matter from a naturally occurring anal orifice and or urine can be provided according to the present invention. Typically such devices comprise a bag (11) having an aperture (21) and a flange (12) surrounding the aperture (21) for adhesive attachment to the perianal area of a wearer as visible from Figure 1.

The bag (11) as used herein is a flexible receptacle for the containment of urine and or excreted faecal matter. The bag (11) can be provided in any shape or size depending on the intended use thereof, i.e. whether the device is intended for bedridden patients or active patients suffering from incontinence or requiring an artificial bowel or for infants. For example, elongated bags which are principally tubular or rectangular are typically utilised by bedridden patients and elderly incontinence sufferers. For more active wearers whether infants or adults, the device should preferably be anatomically shaped such that the device follows the contours of the body and can be worn inconspicuously by the wearer under normal garments.

Particularly, preferred shapes are flat circular type bags, cone shaped bags, truncated shaped bags and pyramidal or truncated pyramidal shaped bags. In a most preferred embodiment of the present invention, the bag (11) has a substantially truncated cone shape. Typically the bags will have a wearer facing portion (16) and a garment facing portion (17). The wearer facing portion (16) of the faecal management device (10) is disposed adjacent the buttocks of the wearer. As such, the wearer facing portion (16) amply covers the buttocks of the wearer and does not hang between the thighs of the wearer.

In addition, the bag (11) is preferably shaped to allow at least partial insertion and retention of the bag in-between the buttocks of the wearer and thereby ensure good contact between the flange and the skin of the wearer. For example, the bag (11) may be provided with a neck portion or conduit.

The bag (11) is preferably designed to provide sufficient volume for faecal material and or urine under a variety of wearing conditions, also when worn by a freely moving, i.e. not bedridden wearer. Sitting on the bag (11), for example, will result in a largely reduced volume in some areas of the bag. Thus, the bag (11) is preferably shaped to provide sufficient volume in areas which are not subjected to much pressure in wearing conditions such as sitting.

The bag (11) is designed to safely contain any entrapped material, typically it will be liquid impermeable, yet it may be breathable. The bag is designed of sufficient strength to withstand rupture in use, also when pressure on the bag is exerted in typical wearing conditions, such as sitting.

According to the present invention, depending on the shape of the bag (11) required, the bag (11) may be provided from a unitary piece of material or from a number of separate pieces of material, which may be identical or different and which are sealed at their respective peripheries.

In one preferred embodiment the bags herein have a wearer facing portion (16) and a garment facing portion (17) which comprise separate pieces of material. The wearer facing portion (16) and the garment facing portion (17) are sealed at the periphery of the bag (11), thus creating a bag peripheral rim (18). As is visible from Figure 1, the wearer facing portion (16) of the bag (11) may comprise two further sections (19), which are secured to each other by means known to the man skilled in the art, such as adhesive, thermobonding or pressure bonding in order to provide the desired bag configuration. Said rim (18) may also be inside the bag, thus being coextensive with the inner surface (15) of the bag (11) rather than with the outer surface (30) of the bag (11). Preferably the bag (11) is asymmetrical to the transversal axis, so that the distance measured in the longitudinal direction from the centre of the aperture (21) to the front end of the bag (11) is shorter than the distance measured to the rear end of the bag (11).

According to the present invention the bag (11) can comprise one or multiple layers, preferably two or three layers. The layer on the inside of the bag, which will typically at least partially come in contact with faecal material is called the inner layer. The outermost layer of the bag, which will typically at least partially come in contact with the skin to the wearer and the garments of the wearer, is called the outer layer.

The layers of the bag material may be provided from any material, preferably so that the bag is liquid impervious. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag is preferably provided with a non-woven layer. Such material layers present an uneven surface to the skin of the wearer and thus reduce significantly the problem of occlusion and greatly improve skin healthiness.

In one preferred embodiment of the present invention the bag comprises two layers. Preferably the outer layer comprises a non-woven layer and the inner layer comprises a film.

In yet another preferred embodiment of the present invention, the bag (11) comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag (11) may be hydrophobic or hydrophilic. If the bag (11) does not comprise a film layer, preferably at least one non-woven layer is hydrophobic. As a consequence, fluid penetration is resisted through the wearer facing portion (16) and the garment facing portion (17) of the faecal management device (10). If the bag comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, non-ionic, cationic and amphoteric surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

In one embodiment of the present invention the bag may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag or may be secured to the inner layer of the bag (11). Any known techniques for securing absorbent material to nonwoven and film substrates may be used to secure the absorbent material to the inner layer of the bag. The absorbent material may also be arranged to have any desired shape or configuration (e.g., rectangular, oval, circular, etc.).

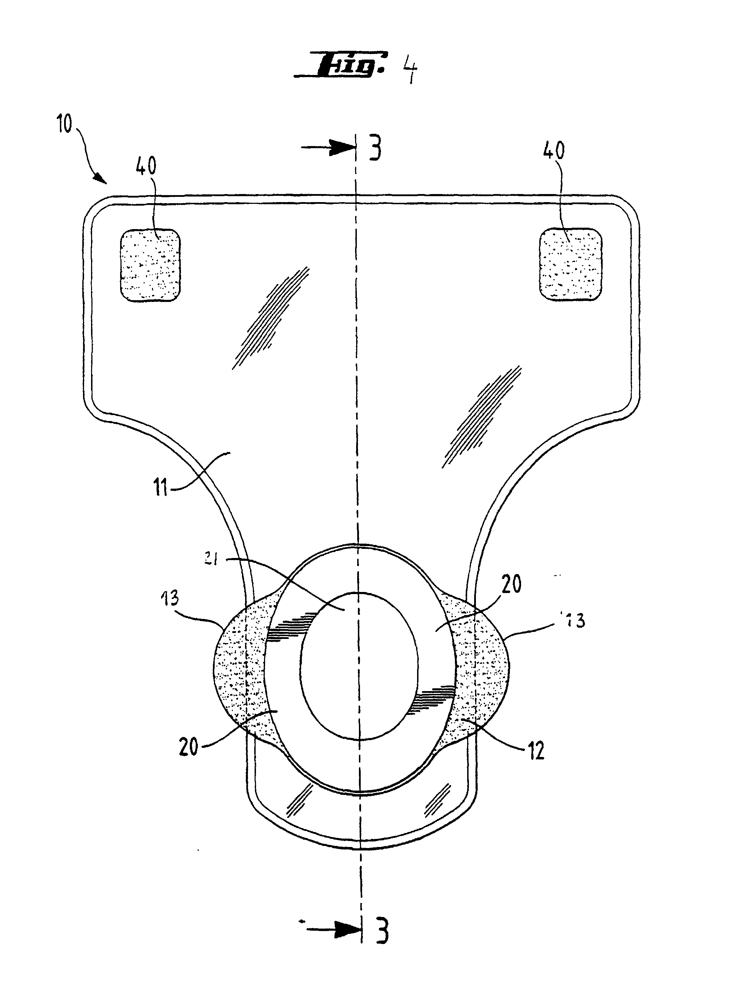

In the embodiment shown in Figure 4, the outer surface of bag (11) is provided with patches of adhesive (40) for securing the bag (11) to the body of the wearer. Preferably, the patches of adhesive (40) are positioned on the outer surface of bag (11) such that they are secured to the abdomen of the wearer in use. Any number, size and shape of adhesive patches (40) may be used depending on the intended use of the device.

The human waste management device in particular urine management devices according to the present invention also preferably comprise an additional acquisition layer. The acquisition layer is typically secured to the inner surface of bag. However, the acquisition layer may also be secured to the flange, or both the flange and the inner surface of bag. The acquisition layer is preferably positioned such that it separates the genitalia of the wearer from coming into direct contact with the absorbent material. The acquisition layer is fluid pervious allowing urine to readily pass through so that it may be absorbed by absorbent material.

The acquisition layer may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. If the acquisition, barrier layer includes fibers, the fibers may be spunbond, carded, wet-laid, meltblown, hydroentangled, or otherwise processed as is known in the art.

The acquisition layer is designed to have a pore size such that the absorbent material is not allowed to pass through and contact the wearer's skin. While designed not to have to large of a pore size which permits the passage of absorbent material, the acquisition layer preferably has a pore size which is greater than the pore size of the absorbent material.

Preferably, the acquisition layer is less hydrophilic than the absorbent material. The acquisition layer may be treated with a surfactant to increase its initial wettability. When treated with surfactant, however, the acquisition layer should still be less hydrophilic than the absorbent material. Suitable methods for treating the acquisition layer with a surfactant include spraying the acquisition layer with the surfactant and immersing the material into the surfactant. Alternatively, a surfactant may be incorporated into the acquisition layer.

As shown in Figure 1 the bag (11) is provided with an aperture (21) whereby faecal matter is received from the body prior to storage within the bag cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction or in both directions, e.g. the contours of the aperture are in the shape of two ellipses with the respective main axes being substantially perpendicular.

The flange (12) is attached to the bag (11) according to any means known to the man skilled in the art which may provide permanent or releasable attachment. Preferably however, the flange is attached to the bag by adhesive. Typically, the bag will be attached to the flange, towards the outer periphery of flange so as not to cause any obstruction for the entering faecal matter.

The flange may be provided in any size depending on the wearer group for which the device is intended. Similarly the flange may be provided in any shape and preferably has a symmetrical shape preferably comprising a plurality of lobes (13).

The flange comprises a garment facing surface (22) and a wearer facing surface (23). In an preferred embodiment these are two large, substantially flat surfaces, however, the flange may also comprise projections (28, 29) designed to fit the perineal or coccygeal area of the wearer.

The flange (12) should be made of soft, flexible and malleable material to allow easy placement of the flange (12) to the perianal area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a basis weight of 5 to 250 g/m², more preferably 50 g/m². Other thermoplastic foam materials, or other suitable plastics sheet materials having the described properties (i.e., softness, pliability, stretchability, and contractability) may also be used. Preferably, the material of garment facing surface (22) of the flange (12) may extend into the defined aperture area so as to form a skirt or flap of material which prevents unintentional adhesion of the surface edges of the flange defining the aperture to oneanother during use.

According to the present invention the faecal management device further comprises an attachment means to secure the device to the wearer. Such means comprises a body-compatible pressure sensitive adhesive (20) applied to the wearer facing surface (23) of the flange (12).

According to the present invention it has now been found that any medically suitable substantially water insoluble pressure sensitive adhesives comprising a polymer which forms a 3-dimensional matrix, and comprises less than 10%, preferably less than 5% by weight of said adhesive of hydrocolloids are particularly affective in providing the desired adhesive properties to secure the flange to the skin of the wearer at the sensitive perineal area even under wet skin conditions, whilst allowing for relatively painless removal after use.

The term hydrocolloid as used herein refers to colloidal absorbent materials, and mixtures of colloidal absorbent materials selected from starch, modified starches such as dextrin, cellulose ester such as carboxymethycellulose, and natural gums such as pectin karaya, gelatin, guar gum, gum arabic, locust bean gum, and carboxypolymethylene.

According to the present invention the 3-dimensional matrix also referred to herein as a gel or hydrogel, comprises as an essential component a polymer which can be physically or chemically cross linked. The polymer may be naturally or synthetically derived. The uncrosslinked polymer includes repeating units derived from vinyl alcohols, vinyl ethers and their copolymers, carboxy vinyl monomer, vinyl ester monomers, esters of carboxy vinyl monomers, vinyl amide monomers, hydroxy vinyl monomers, cationic vinyl monomers containing amines or quaternary groups, N-vinyl lactam monomer, polyethylene oxides, polyvinylpyrrolidon (PVP), acrylics such as hydroxyethylmethacrylate, methoxydiethoxyethyl methacrylate and hydroxydiethoxyethyl methacrylate and sulphonated polymers such as acrylamide sulphonated polymers and mixtures thereof. Alternatively, the uncrosslinked polymer may be a homopolymer or copolymer of a polyvinyl ether, or a copolymer derived from half ester of maleic ester. Similarly any other compatible polymer monomer units may be used as copolymers such as for example polyvinyl alcohol and polyacrylic acid or ethylene and vinyl acetate.

As another alternative, the polymers may be block copolymer thermoplastic elastomers such as ABA block copolymers such as styrene-olefin-styrene block copolymers or ethylene-propylene block copolymers. More preferably such polymers include hydrogenated grade Styrol/Ethylene-Butylene/Styrol (SEBS), Styrene/Isoprene/Styrene (SIS), and Styrol/Ethylene-Propylene/Styrol (SEPS).

Particularly preferred polymers are acrylics, sulphonated polymers such as acrylamide sulphonated polymers, vinyl alcohols, vinyl pyrrolidine, polyethylene oxide and mixtures thereof.

According to the present invention the 3 dimensional adhesive matrix also essentially comprises a plasticiser, which is preferably a liquid at room temperature. This material is selected such that the polymer may be solubilized or dispersed within the plasticiser. For embodiments wherein irradiation cross linking is to be carried out, the plasticiser must also be irradiation cross linking compatible such that it does not inhibit the irradiation cross linking process of the polymer. The plasticiser may be hydrophilic or hydrophobic.

Suitable plasticisers include water, alcohols, polyhydric alcohols such as glycerol and sorbitol, and glycols and ether glycols such as mono- or diethers of polyalkylene gylcol, mono- or diester polyalkylene glycols, polyethylene glycols (typically up to a molecular weight of about 600), glycolates, glyceril, sorbitan esters, esters of citric and tartaric acid, imidazoline derived amphoteric surfactants, lactams, amides, polyamides, quaternary ammonium compounds, condensation products of polyethylene imine and epichlorohydrin, liquid polybutenes, esters such phthalates, adipates, stearates, palmitates, sebacates, or myristates, natural or synthetic oils such as vegetable oils, mineral oils, and combinations thereof. Particularly preferred are polyhydric alcohols, polyethylene glycol (with a molecular weight up to about 600), glycerol, sorbitol, water and mixtures thereof.

Typically the adhesive comprises a ratio of polymer to plasticiser by weight of from 1:100 to 100:1, more preferably from 50:1 to 1:50. However, the exact amounts and ratios of the polymer and plasticiser will depend to a large extent on the exact nature of polymer and plasticisers utilised and can be readily selected by the skilled person in the art. For example a high molecular weight polymer material will require a greater amount of plasticiser than a low molecular weight polymer.

In addition to the polymer and plastisicer components of the adhesive, the adhesive may comprise a number of optional additional components for example the composition may comprise from 0% to 50% by weight of the composition, of a tackifying resin Such tackifying resins are particularly useful in combination with ABA block copolymer adhesive compositions. Suitable tackifying resins include for example rosin derivatives terpene, terpene-phenolic resins, hydrocarbon resins such as C₅ and C₅/C₉ resins, aromatic resins and hydrogenated resins.

Other suitable optional ingredients include from 0% to 10% and more preferably form 0% to 5% by weight of substances for further facilitating and stabilising the 3-dimensional matrix and the matrix forming process. For example for hydrophobic adhesive compositions these may be fatty acids of C₈ to C₂₂, their metallic salts and their polyoxo-derivatives; lanolin derivatives; silica; bentonite, montmorillonite and their derivatives; waxes or mixtures thereof.

Other common additives known in the art such as preservatives, antioxidants, anti UV agents, pigments, mineral fillers and mixtures thereof may also be comprised within the adhesive composition in quantities up to 10% each respectively.

According to the present invention the polymer component of the adhesive can be physically or chemically cross linked in order to form the 3-dimensional matrix. Physical cross linking refers to polymers having cross links which are not chemical covalent bonds but are of a physical nature such that there are areas in the 3-dimensional matrix having high crystallinity or areas having a high glass transition temperature. Chemical cross linking refers to polymers which are linked by chemical bonds. Preferably the polymer is chemically cross linked by radiation techniques such as thermal-, E beam-, UV-, gamma or micro-wave radiation.

In addition when chemical crosslinks are formed in the system, a polyfunctional crosslinker and/or a free radical initiator may be present in the premix to initiate the crosslinking upon irradiation. Such components can be present preferably in quantities up to 5% by weight.

The resulting adhesive compositions may be divided into three family types; hydrophilic, hydrophobic and mixed phase compositions dependant upon the 5 nature of the components of the adhesive.

Hydrophilic adhesives are compositions in which typically the plasticiser is water or glycerol or glycol and/or mixtures thereof and the polymeric phase is of synthetic (e.g. polyacrylics). Optionally such compositions may comprise up to 10% by weight of colloid natural gums.

Hydrophobic adhesives are compositions in which the plasticiser is typically an oil or blend of oils of vegetable or mineral origin and the polymer is usually a synthetic polymer, preferably an elastomer, which is soluble or dispersible in such oils.

Mixed phase adhesives are compositions in which both hydrophobic and hydrophilic components, possibly in both plasticisers and polymers, form two or more separate phases. In such cases an emulsifier is preferably present at a suitable level to form stable emulsions between the incompatible phases.

The preferred adhesive compositions for use in the present invention are hydrophilic, which provide particularly satisfactory adhesion on wet skin.

Suitable adhesives for use herein include Promeon, available from Promeon Division of Medtronic Inc., Minneapolis Minnesota, USA and hydrogel adhesive available from 3M.

The adhesive (20) can be applied to the wearer facing surface (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for faecal management devices to be used for babies the amount of adhesive may be less than for faecal management devices designed for active adult incontinence sufferers.

The adhesive (20) is preferably covered with a release means (not shown) in order to protect the adhesive (20), such as siliconized paper. The adhesive (20) can cover the entire wearer facing surface of the flange or more preferably have at least one, preferably two to six non-adhesive portions. These portions may be adhesive free or may contain inactivated or covered adhesives. As is evident from Figure 4, the adhesive is in one preferred embodiment not applied to the entire wearer facing surface area of the flange (12), so as to provide lobes (13) on either side of the flange (12) which are non-adhesive and can thereby serve to facilitate placement and removal of the device whilst avoiding contact with the adhesive. These lobes are however preferably also covered by the release means. Before application of the faecal management device (10) to the skin of the wearer, the release means if present is removed.

### Detailed description of a diaper to be worn in combination with the human waste management device

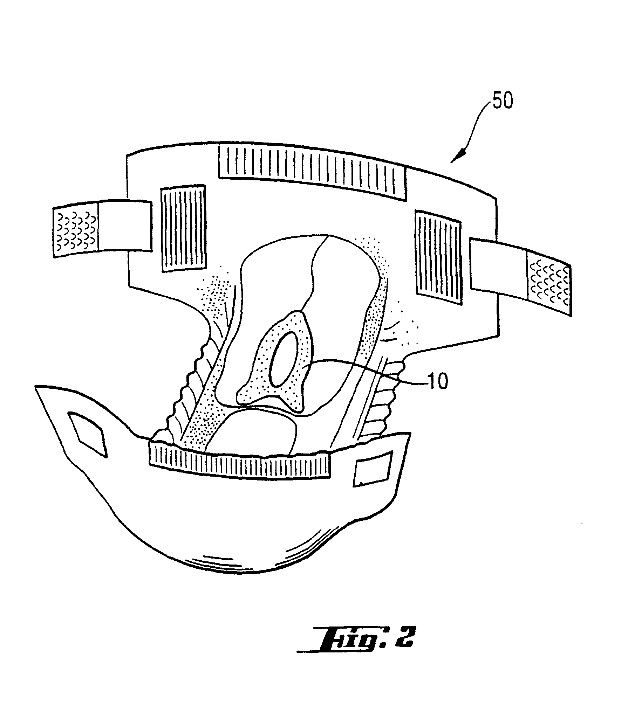

The human waste management devices, in particular the faecal management device (10) of the present invention has been found to be particularly useful and beneficial when used in conjunction with a garment, or diaper (50), preferably a disposable diaper - refer to Figure 2. The faecal management device (10) is preferably first positioned in the perianal area of the wearer before the disposable diaper (50) is applied. In particular, the diaper (50) is positioned over the faecal management device (10) and fastened in a conventional manner around the body of the wearer. It has been found that, in addition, to providing excellent separation between urine and faecal material, the combined faecal management device (10) and diaper (50) system actually reduces skin irritation, which may at times occur, especially since the group of typical wearers includes the very old, the very young and the unhealthy wearers. In effect, the presence of the faecal management device (10) permits the formation of a separation layer between the skin of the wearer and the diaper (50), i.e. a part of the absorbent core (58) of the diaper (10). The diaper (50) can be of the conventional type (an embodiment of which is described below although not a limiting example by any means) or can be adapted to contain in an effective and comfortable manner the faecal management device (10) according to the teachings of the present invention.

As used herein, the term "disposable diapers" refers to articles which absorb and contain body extrudates; and more specifically, refers to articles which are placed against or in proximity to the body of the wearer to absorb and contain the various extrudates discharged from the body and which are intended to be discarded after a single use (i. e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer.

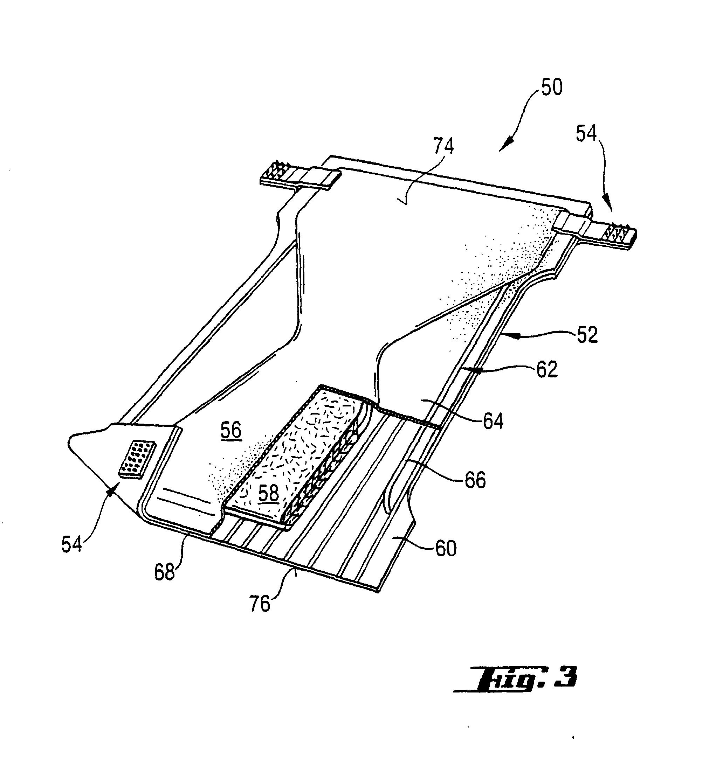

Figure 3 is a partially cut-away perspective view of a diaper (50) embodying the present invention prior to it being placed on the wearer over the faecal management device (10). As is visible from Figure 3, a preferred diaper (50) comprises a body portion (52) and a refastenable mechanical fastening device (54). A preferred body portion (52) comprises a liquid pervious topsheet (56), and absorbent core (58), a liquid impervious backsheet (60), and elastically contractible leg cuffs (62); each leg cuff (62) preferably comprising a side flap (64) and one or more elastic members (66). For simplicity purposes, only one elastic member (66) is shown in the side flap (64). While the topsheet (56), the absorbent core (58), the backsheet (60), the side flaps (64), and the elastic members (66) may be assembled in a variety of well-known configurations. A preferred disposable diaper configuration is shown and generally described in US 3,860,003, an even more preferred disposable diaper configuration is shown and generally described in WO 93/16669. In this preferred diaper configuration, the backsheet (60) is joined to the topsheet (56); the absorbent core (58) is positioned between the topsheet (56) and the backsheet (60); the side flaps (64) extend outwardly from and along each side edge of the absorbent core (58); and the elastic member (66) is operatively associated with each side flap (64).

Figure 3 shows the body portion (52) in which the topsheet (56) and the backsheet (60) are coextensive and have length and width dimensions generally larger than those of the absorbent core (58). The topsheet (56) is superposed on the backsheet (60) thereby forming the periphery (68) of the body portion (52).

The body portion (52) has an inside surface (74) and an outside surface (76). When a backsheet (60) is used, it typically forms the outside surface (76) of the body portion (52). The inside surface (74) is that surface of the diaper (50) opposite the outside surface (76) and in the embodiment shown is typically formed by the topsheet (56). In general, the inside surface (74) of the diaper (50) is that surface coextensive with the outside surface (76) and which is for the greater part in contact with the wearer when the diaper (50) is worn.

The absorbent core (58) of the body portion (52) may be any absorbent means which is generally compressible, conformable, non-irritating to the skin of the wearer, and capable of absorbing and retaining liquids such as urine and other certain bodily discharges. The absorbent core (58) may be manufactured in a variety of sizes and shapes (for example, rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core (58) may also be varied (for example, the absorbent core (58) may have varying caliper zones, hydrophilic gradients, superabsorbent gradients, or lower average density and lower average basis weight acquisition zones; or may comprise one or more layers or structures). Further, the size and absorbent capacity of the absorbent core (58) may be varied to accommodate wearers ranging from infants to adults.

The backsheet (60) is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet (60) prevents the exudates absorbed and contained in the absorbent core (58) from soiling articles which are in contact with the diaper (50) such as undergarments and bedding. The backsheet (60) may thus comprise polymeric films such as thermoplastic films of polyethylene or polypropylene, or composite materials such as film-coated non-woven material. Exemplary films are manufactured by Tredegar Industries, Inc. of Terre Haute, Ind., USA or BP-Chemical PlasTec, Rotbuchenstrasse 1, D-8000 München, Germany.

The backsheet (60) is preferably textured to provide a more clothlike appearance. Further, the backsheet (60) may also permit vapours to escape from the absorbent core (58) while still preventing exudates from passing through the backsheet (60) by, for example, being supplied with microapertures. The size of the backsheet (60) is dictated by the size of the absorbent core (58) and the exact diaper design selected.

The topsheet (56) of the diaper is compliant, soft feeling and non-irritating to the skin of the wearer. Further, the topsheet (56) is liquid pervious permitting liquids (for example, urine) to readily penetrate through its thickness. A suitable topsheet (56) may be manufactured from a wide range of materials, such as porous foams, reticulated foams, apertured films; or woven or non-woven webs of natural fibres (for example, wood or cotton fibres) or from a combination of natural and synthetic fibres. Preferably, it is made of a material that isolates the skin of the wearer from liquids retained in the absorbent core (58).

There are a number of manufacturing techniques which may be used to manufacture the topsheet (56). For example, the topsheet (56) may be a non-woven web of fibres. An exemplary topsheet (56) is carded and thermally bonded by means well-known to those skilled in the fabric art. A suitable topsheet (56) is manufactured by, for example, Veratec Inc., a division of International Paper Company, of Walpole, Mass., USA. A topsheet (56) particularly preferred for incontinence garments comprises a formed thermoplastic film.

## Claims

1. A disposable human waste management device (10) comprising a bag (11), said bag (11) having an aperture and a flange (12) surrounding said aperture (21) said flange having a wearer facing surface (23) and a garment facing surface (22), wherein said wearer facing surface (23) comprises an adhesive (20) for adhesive attachment to the perianal area of wearer, **characterised in that** said adhesive (20) is a substantially water insoluble pressure sensitive adhesive comprising a polymer which forms a 3-dimensional matrix by being crosslinked, and comprises less than 10% hydrocolloids, wherein said polymer is either physically crosslinked, or chemically crosslinked by radiation techniques consisting of thermal-, or UV-, or micro-wave radiation.

2. A disposable human waste management device (10) comprising a bag (11), said bag (11) having an aperture and a flange (12) surrounding said aperture (21) said flange having a wearer facing surface (23) and a garment facing surface (22), wherein said wearer facing surface (23) comprises an adhesive (20) for adhesive attachment to the perianal area of wearer, **characterised in that** said adhesive (20) is a substantially water insoluble pressure sensitive adhesive comprising a polymer which forms a 3-dimensional matrix by being crosslinked, and comprises less than 10% hydrocolloids, wherein said polymer is chemically crosslinked and wherein a polyfunctional crosslinker and/or a free radical initiator are present in the premix when chemical crosslinks are formed.

3. A disposable human waste management device (10) according to claim 1, wherein when chemical crosslinks are formed, a polyfunctional crosslinker and/or a free radical initiator are present in the premix.

4. A disposable human waste management device (10) according to claim 2 or 3, wherein said polyfunctional crosslinker and/or said free radical initiator are present in quantities up to 5% by weight.

5. A disposable human waste management device (10) according to claim 1 or 2, wherein said adhesive comprises less than 5% by weight of said adhesive of hydrocolloids.

6. A disposable human waste management device (10) according to claim 1 or 2, wherein said polymer is selected from acrylics, sulphonated polymers, vinyl alcohols, vinyl pyrrolidine, polyethylene oxide or mixtures thereof.

7. A disposable human waste management device (10) according to claim 1 or 2, wherein said adhesive (20) further comprises a plasticiser.

8. A disposable human waste management device (10) according to claim 7, wherein said plasticizer is selected from polyhydric alcohols, polyethylene glycols, glycerol, sortbitol, water or mixtures thereof.

9. A disposable human waste management device (10) according to claim 1 or 2, wherein said adhesive is hydrophilic, or a hydrophilic-hydrophobic mixed phase adhesive.

10. A disposable human waste management device (10) according to any one of the preceding claims, wherein said wearer facing surface (23) of said flange (12) comprises from 20 g/m² to 2500 g/m² of said adhesive (20).

11. A disposable human waste management device (10) according to claim 10, wherein said wearer facing surface (23) of said flange (12) comprises from 500 g/m² to 2000 g/m² of said adhesive (20), preferably from 700 g/m² to 1500 g/m² of said adhesive (20).

12. A disposable human waste management device (10) according to any of the preceding claims, wherein said wearer facing surface (23) of said flange (12) comprises at least one non adhesive portion (13).

13. A disposable human waste management device (10) according to any one of the preceding claims, wherein said adhesive is applied to said wearer facing surface (23) of said flange (12) by slot coating.

14. A disposable human waste management device (10) according to any one of the preceding claims, wherein said bag (11) has a substantially truncated cone shape.

15. The use of a disposable human waste management device (10) according to any of the preceding claims in combination with a disposable diaper (50).

16. The use of a disposable human waste management device (10) according to claim 15 wherein said disposable human waste management device is a faecal management device (10), whereby said faecal management device (10) is first positioned in between the buttock cheeks of the wearer and then said disposable diaper (50) is positioned over said faecal management device (10) and fastened in a conventional manner around the trunk of said wearer.

## Patentansprüche

1. Wegwerfbare Managementvorrichtung (10) für menschliche Ausscheidungen mit einer Tasche (11), wobei die Tasche (11) eine Öffnung und einen die Öffnung (21) umgebenden Flansch (12) aufweist, wobei der Flansch eine trägerseitige Oberfläche (23) und eine wäscheseitige Oberfläche (22) aufweist, wobei die trägerseitige Oberfläche (23) ein Haftmittel (20) zur Haftanbringung des perianalen Bereichs des Trägers umfaßt, **dadurch gekennzeichnet, daß** das Haftmittel (20) ein im wesentlichen wasserlösliches, druckempfindliches Haftmittel ist, das ein Polymer aufweist, welches durch Vernetzung eine dreidimensionale Matrix bildet und weniger als 10% Hydrokolloide aufweist, wobei das Polymer durch Strahlungstechniken bestehend aus thermischer, UV- oder Mikrowellen-Strahlung physikalisch vernetzt oder chemisch vernetzt ist.

2. Wegwerfbare Managementvorrichtung (10) für menschliche Ausscheidungen mit einer Tasche (11), wobei die Tasche (119 einen Öffnung und einen die Öffnung (21) umgebenden Flansch (12) aufweist, wobei der Flansch eine trägerseitige Oberfläche (23) und eine wäscheseitige Oberfläche (22) aufweist, wobei die trägerseitige Oberfläche (23) ein Haftmittel (20) zur Haftanbringung an das perianale Gebiet des Trägers umfaßt, **dadurch gekennzeichnet, daß** das Haftmittel (20) ein im wesentlichen wasserunlösliches, druckempfindliches Haftmittel ist, das ein Polymer aufweist, welches durch Vernetzung eine dreidimensionale Matrix bildet, und weniger als 10% Hydrokolloide umfaßt, wobei das Polymer chemisch vernetzt ist und wobei ein polyfunktionaler Vernetzer und/oder ein Freies-Radikal-Initiator in der Premix vorhanden ist, wenn die chemischen Vernetzungen gebildet werden.

3. Wegwerfbare Managementvorrichtung (10) für menschliche Ausscheidungen nach Anspruch 1, in welcher, wenn chemische Vernetzungen gebildet werden, ein polyfunktionaler Vernetzer und/oder ein Freies-Radiakal-Initiator in der Premix vorhanden sind.

4. Wegwerfbare Managementvorrichtung (10) für menschliche Ausscheidungen gemäß Anspruch 2 oder 3, in welcher der polyfunktionaler Vernetzer und/oder der Freies-Radikal-Initiator in Mengen von bis zu 5 Gew.% vorhanden ist.

5. Wegwerfbare Managementvorrichtung (10) für menschliche Ausscheidungen gemäß Anspruch 1 oder 2, in welcher das Haftmittel weniger als 5 Gew.% des Haftmittels von Hydrokolloiden umfaßt.

6. Wegwerfbare Managementvorrichtung (10) fiir menschliche Ausscheidungen gemäß Anspruch 1 oder 2, in welcher das Polymer ausgewählt ist aus Acrylen, sulfonisierten Polymeren, Vinylalkoholen, Vinylpyrolidin, Polyethylenoxid oder Mischungen davon.

7. Wegwerfbare Managementvorrichtung (10) für menschliche Ausscheidungen gemäß Anspruch 1 oder 2, in welcher das Haftmittel (20) ferner einen Weichmacher umfaßt.

8. Wegwerfbare Managementvorrichtung (10) für menschliche Ausscheidungen gemäß Anspruch 7, in welcher der Weichmacher ausgewählt ist aus mehrwertigen Alkoholen, Polyethylenglycolen, Glycerol, Sorbitol, Wasser oder Mischungen davon.

9. Wegwerfbare Managementvorrichtung (10) für menschliche Ausscheidungen gemäß Anspruch 1 oder 2, in welcher das Haftmittel hydrophil ist oder ein Haftmittel mit einer hydrophilen-hydrophoben Mischphase ist.

10. Wegwerfbare Managementvorrichtung (10) für menschliche Ausscheidungen gemäß einem der vorstehenden Ansprüche, in welcher die trägerseitige Oberfläche (23) des Flansches (12) von 20 g/m² bis 2500 g/m² des Haftmittels (20) umfaßt.

11. Wegwerfbare Managementvorrichtung (10) für menschliche Ausscheidungen gemäß Anspruch 10, in welcher die trägerseitige Oberfläche (23) des Flansches (12) von 500 g/m² bis 2000 g/m² des Haftmittels (20), vorzugsweise von 700 g/m² bis 1500 g/m² des Haftmittels (20) umfaßt.

12. Wegwerfbare Managementvorrichtung (10) fiir menschliche Ausscheidungen gemäß einem der vorstehenden Ansprüche, in welcher die trägerseitige Oberfläche (23) des Flansches (12) wenigstens einen nicht haftenden Bereich (13) umfaßt.

13. Wegwerfbare Managementvorrichtung (10) für menschliche Ausscheidungen gemäß einem der vorstehenden Ansprüche, in welcher das Haftmittel aufgebracht ist auf die trägerseitige Oberfläche (23) des Flansches (12) durch Schlitzbeschichtung.

14. Wegwerfbare Managementvorrichtung (10) für menschliche Ausscheidungen gemäß einem der vorstehenden Ansprüche, in welcher die Tasche (11) eine im wesentlichen kegelstumpfförmige Gestalt hat.

15. Verwendung einer wegwerfbaren Managementvorrichtung (10) für menschliche Ausscheidungen gemäß einem der vorstehenden Ansprüche in Kombination mit einer Einwegwindel (50).

16. Verwendung der wegwerfbaren Managementvorrichtung (10) für menschliche Ausscheidungen gemäß Anspruch 15, bei welcher die wegwerfbare Managementvorrichtung für menschliche Ausscheidungen eine Fäkal-Managementvorrichtung (10) ist, wobei die Fäkal-Managementvorrichtung (10) zuerst zwischen den Gesäßhälften des Trägers positioniert wird und dann die Einwegwindel (50) über der Fäkal-Managementvorrichtung (10) positioniert und in einer herkömmlichen Weise um den Rumpf des Trägers herum befestigt wird.

## Revendications

1. Dispositif jetable (10) pour maîtriser les rejets humains, qui comprend un sac (11), ledit sac étant muni d'une ouverture et d'une collerette (12) entourant ladite ouverture (21), ladite collerette ayant une surface (23) tournée vers le porteur et une surface (22) tournée vers le vêtement, dans lequel ladite surface (23) tournée vers le porteur comprend un adhésif (20) destiné à l'attache adhésive à la zone périnéale du porteur, **caractérisé en ce que** ledit adhésif (20) est un adhésif pratiquement insoluble dans l'eau et sensible à la pression, comprenant un polymère qui forme une matrice tri-dimensionnelle en étant réticulé et qui comprend moins de 10 % d'hydrocolloïdes, dans lequel ledit polymère est soit réticulé physiquement, soit réticulé chimiquement, par des techniques de radiation qui consistent en des rayonnements thermiques, UV ou de micro-ondes.

2. Dispositif jetable (10) pour maîtriser les rejets humains, qui comprend un sac (11), ledit sac (11) étant muni d'une ouverture et d'une collerette (12) entourant ladite ouverture (21), ladite collerette ayant une surface (23) tournée vers le porteur et une surface (22) tournée vers le vêtement, dans lequel ladite surface (23) tournée vers le porteur comprend un adhésif (20) destiné à l'attache adhésive à la zone périnéale du porteur, **caractérisé en ce que** ledit adhésif (20) est un adhésif pratiquement insoluble dans l'eau et sensible à la pression, comprenant un polymère qui forme une matrice tri-dimensionnelle en étant réticulé et qui comprend moins de 10 % d'hydrocolloïdes, dans lequel ledit polymère est réticulé chimiquement et dans lequel un réticulant polyfonctionnel et/ou un initiateur de radicaux libres sont présents dans le prémix lorsque des réticulations chimiques se forment.

3. Dispositif jetable (10) pour maîtriser les rejets humains selon la revendication 1, dans lequel, lorsque des réticulations chimiques se forment, un réticulant polyfonctionnel et/ou un initiateur de radicaux libres sont présents dans le prémix.

4. Dispositif jetable (10) pour maîtriser les rejets humains selon la revendication 2 ou 3, dans lequel ledit réticulant polyfonctionnel et/ou ledit initiateur de radicaux libres sont présents en quantités allant jusqu'à 5 % en poids.

5. Dispositif jetable (10) pour maîtriser les rejets humains selon la revendication 1 ou 2, dans lequel ledit adhésif constitue moins de 5 % en poids dudit adhésif d'hydrocolloïdes.

6. Dispositif jetable (10) pour maîtriser les rejets humains selon la revendication 1 ou 2, dans lequel ledit polymère est sélectionné parmi des acryliques, des polymères sulfonés, des alcools vinyliques, la vinylpyrrolidine, l'oxyde de polyéthylène ou leurs mélanges.

7. Dispositif jetable (10) pour maîtriser les rejets humains selon la revendication 1 ou 2, dans lequel ledit adhésif (20) contient en outre un plastifiant.

8. Dispositif jetable (10) pour maîtriser les rejets humains selon la revendication 7, dans lequel ledit plastifiant est sélectionné parmi des polyalcools, des polyéthylèneglycols, le glycérol, le sorbitol, l'eau ou leurs mélanges.

9. Dispositif jetable (10) pour maîtriser les rejets humains selon la revendication 1 ou 2, dans lequel ledit adhésif est hydrophile ou un adhésif à phase mixte hydrophile-hydrophobe.

10. Dispositif jetable (10) pour maîtriser les rejets humains selon l'une quelconque des revendications précédentes, dans lequel ladite surface (23), tournée vers le porteur, de ladite collerette (12), contient de 20 g/m² à 2 500 g/m² dudit adhésif (20).

11. Dispositif jetable (10) pour maîtriser les rejets humains selon la revendication 10, dans lequel ladite surface (23), tournée vers le porteur, de ladite collerette (12), contient de 500 g/m² à 2 000 g/m² dudit adhésif (20), de préférence de 700 g/m² à 1 500 g/m² dudit adhésif (20).

12. Dispositif jetable (10) pour maîtriser les rejets humains selon l'une quelconque des revendications précédentes, dans lequel ladite surface (23) tournée vers le porteur, de ladite collerette (12), contient au moins une partie non adhésive (13).

13. Dispositif jetable (10) pour maîtriser les rejets humains selon l'une quelconque des revendications précédentes, dans lequel ledit adhésif est appliqué à ladite surface (23), tournée vers le porteur, de ladite collerette (12) par enduction à travers une fente.

14. Dispositif jetable (10) pour maîtriser les rejets humains selon l'une quelconque des revendications précédentes, dans lequel ledit sac (11) a sensiblement une forme de cône tronqué.

15. Utilisation d'un dispositif (10) pour maîtriser les rejets humains selon l'une quelconque des revendications précédentes en combinaison avec une couche-culotte jetable (50).

16. Utilisation d'un dispositif (10) pour maîtriser les rejets humains selon la revendication 15, dans laquelle ledit dispositif pour maîtriser les rejets humains est un dispositif (10) de maîtrise fécale, dans laquelle ledit dispositif de maîtrise fécale est d'abord placé entre les fesses du porteur puis ladite couche-culotte jetable (50) est placée sur ledit dispositif (10) de maîtrise fécale et attachée de manière habituelle autour du tronc dudit porteur.
